# EUROPEAN PATENT SPECIFICATION

(11) **EP 1 596 886 B1**
(45) Date of publication and mention of the grant of the patent: **16.12.2009**
(21) Application number: 04715336.6
(22) Date of filing: 27.02.2004
(51) Int. Cl.: A61K 41/00

(54) **ACTIVATION AND PRODUCTION OF RADIOLABELED PARTICLES**
AKTIVIERUNG UND HERSTELLUNG VON RADIOMARKIERTEN PARTIKELN
ACTIVATION ET PRODUCTION DE PARTICULES RADIOMARQUEES

(30) Priority: 28.02.2003 EP 03100508
(43) Date of publication of application: 23.11.2005
(73) Proprietor: The European Atomic Energy Community (EURATOM), represented by the European Commission, 1049 Brussels (BE)
(72) Inventor: MAGILL, Joseph, 76139 Karlsruhe (DE); GALY, Jean, 76131 Karlsruhe (DE); APOSTOLIDIS, Christos, 69119 Heidelberg (DE); SOMERS, Joseph, 76227 Karlsruhe (DE); JEHENSON, Philippe, F-75014 Paris (FR)
(74) Representative: Office Freylinger
(86) International application number: PCT/EP2004/050222
(87) International publication number: WO 2004/075819

(56) References cited:
- EP-A- 1 162 626
- EP-A- 1 162 626
- EP-A- 1 321 948
- WO-A-00/29034
- WO-A-86/03124
- WO-A-86/03124
- DE-A- 10 037 439
- DE-A- 10 037 439
- US-A- 5 342 283
- LEEMANS W P ET AL: "Radio-isotope production using laser wakefield accelerators" PROCEEDINGS OF THE 2001 PARTICLE ACCELERATOR CONFERENCE. PAC 2001. CHICAGO, IL, JUNE 18 - 22, 2001, PARTICLE ACCELERATOR CONFERENCE, NEW YORK, NY: IEEE, US, vol. 1 OF 5, 18 June 2001 (2001-06-18), pages 129-131, XP010582373 ISBN: 0-7803-7191-7
- PATENT ABSTRACTS OF JAPAN vol. 017, no. 647 (C-1135), 2 December 1993 (1993-12-02) & JP 05 208919 A (ION KOGAKU SHINKO ZAIDAN), 20 August 1993 (1993-08-20)
- PATENT ABSTRACTS OF JAPAN vol. 1995, no. 11, 26 December 1995 (1995-12-26) & JP 07 222804 A (ION KOGAKU SHINKO ZAIDAN), 22 August 1995 (1995-08-22)
- LEEMANS W P ET AL: "Radio-isotope production using laser wakefield accelerators" PROCEEDINGS OF THE 2001 PARTICLE ACCELERATOR CONFERENCE. PAC 2001. CHICAGO, IL, JUNE 18 - 22, 2001, PARTICLE ACCELERATOR CONFERENCE, NEW YORK, NY: IEEE, US, vol. 1 OF 5, 18 June 2001 (2001-06-18), pages 129-131, XP010582373 ISBN: 0-7803-7191-7
- PATENT ABSTRACTS OF JAPAN vol. 017, no. 647 (C-1135), 2 December 1993 (1993-12-02) & JP 05 208919 A (ION KOGAKU SHINKO ZAIDAN), 20 August 1993 (1993-08-20)
- PATENT ABSTRACTS OF JAPAN vol. 1995, no. 11, 26 December 1995 (1995-12-26) & JP 07 222804 A (ION KOGAKU SHINKO ZAIDAN), 22 August 1995 (1995-08-22)
- R. SELWYN ET AL: "A new positron-emitting microsphere to assess the in vivo distribution and to improve the dosimetry of 90Y SIR-spheres in selective internal radiation therapy (SIRT): a microPET-study" J NUCL MED, vol. 48, 2007, page 308P,
- Retrieved from the Internet: URL:http://www.radiologie-lmu.de/de/patien teninformationen/selektive-interne-radio-t herapie.html>

## Description

### FIELD OF THE INVENTION

The present invention generally relates to the activation and production of radiolabeled particles for internal radiopharmaceutical use.

### BACKGROUND OF THE INVENTION

Radiolabeled particles are currently used for two main radiopharmaceutical applications: internal radiation therapy and nuclear medical imaging.

As is well known, such radiolabeled particles generally consist of radionuclides combined with carrier materials. Their dimensions vary from a few nanometers to several hundreds of micrometers and the particles are generally of spherical form. Therefore, they are often referred to as microparticles, microspheres, nanoparticles, beads or microcapsules.

Many different types of nano- and microparticles have been developed.
The carrier material may be based on polymers, polymeric resins, albumin, or inorganic materials such as e.g. glass. The choice of radionuclide depends on the intended use of the radiolabeled particle. For radiotherapy, the radionuclide must have an appropriate radiation spectrum for treating small to large multiple tumours. Generally, β- emitters are used. For nuclear medical imaging, the radionuclide should be a β+ emitter, so that γ-rays produced by the annihilation process can be detected for imaging purposes

The preparation of radiolabeled micro- and nanoparticles has been widely described in the scientific and patent literature. In most methods, particles comprising stable precursor nuclides combined with carrier material are prepared, and the particles are subsequently activated in a nuclear reactor by neutron bombardment In some methods, the radionuclides are first produced, and particles are then formed by combining the radionuclides with the carrier material.

A disadvantage of these methods is that they require a nuclear reactor for activating the precursor nuclide into the radioisotope suitable for radiotherapy or imaging.

DE 10037439 describes a method for activating iodine, wherein iodine-127 is irradiated by neutrons. The irradiating neutrons are generated by a moderator material, itself excited by a neutron emitted by Berrillium material under irradiation by photons produced by a laser beam impinging onto a tantalum wire.

JP 05-208919, JP 07-222804, WO86/03 and US 5,342,283 describe activation of microspheres, e.g. glass microspheres, by neutron irradiation

### OBJECT OF THE INVENTION

The object of the present invention is to provide an alternative way of preparing radiolabeled particles for radiopharmaceutical use. This object is achieved by a method as claimed in claim 1.

### SUMMARY OF THE INVENTION

According to one aspect, the present invention proposes a method for activating particles for internal radiopharmaceutical use, the particles comprising precursor nuclides to be activated. According to the invention, the method comprises:
directing a high-intensity laser beam onto a converting means to produce an irradiating field of bremsstrahlung photons; and
irradiating the particles comprising precursor nuclides in the irradiating field to activate the precursor nuclides, thereby obtaining radiolabeled particles.

It will be appreciated that the present method uses a laser to produce the irradiating field that will induce the nuclear reactions required to activate the precursor nuclides, This eliminates the need for a nuclear reactor. The interaction of the high-intensity laser beam with the converting means produces a high-energy irradiating field of photons. The use of a laser beam for activating particles such as microspheres and nanoparticles instead of a nuclear reactor proves extremely advantageous, in terms of cost, size, operation and maintenance. So-called tabletop-lasers are very compact and are particularly suitable for installation in hospitals. The present method can thus easily be implemented in hospitals or other radiotherapy treatment centers, without relying on distant nuclear reactors.

The irradiating field is a bremsstrahlung photon field. Therefore, the converting means preferably includes a solid target, such as e.g. a metallic foil. More preferably, the converting means includes a first target part on which the laser beam is focused, thereby creating high-energy electrons. A second target part is placed behind the first target and the high-energy electrons impinge thereon, so that it acts as a bremsstrahlung converter creating high-energy photons. The production of photons thus permits the induction of photonuclear reactions (noted (γ, n)) in the particles. Absorption of high-energy electromagnetic radiation in the form of gamma-ray photons-produced by the interaction of the laser beam with the converting means-causes a precursor nuclide to eject a neutron, resulting in the formation of a radioactive isotope of the same element.

Preferred materials for the metallic target, respectively first and second target parts, are tantalum, tungsten, platinum or copper.

The present method allows activation of any particles suitable for radiopharmaceutical use, which comprise precursor nuclides that can be activated through (γ, n) reactions. In particular, the method allows activation of particles comprising as precursor nuclides a stable isotope of an element from the following list: Ag, Au, Br, C, Cd, Ce, Cl, Cr, Cu, Er, Eu, F, Fe, Ga, Gd, Ge, l, In, lr, K, Kr, Lu, Mo, N, Nd, Ni, O, Os, P, Pd, Pr, Pt, Rb, Re, Ru, Sb, Sc, Se, Sm, Sn, Te, Ti, W, Xe, Yb, Zn. Irradiating stable isotopes of these elements with photons will thus produce radionuclides having radiation spectra appropriate for medical use.

For cancer therapy, particles labelled with a β- emitting radionuclide are preferred. A preferred list of suitable radionuclides is the following: ⁷⁰Ga, ⁷⁵Ge, ⁸⁰Br, ⁸¹Se, ^{81m}Se, ^{85m}Kr, ⁸⁶Rb, ⁹⁹Mo, ¹⁰³Ru, ¹⁰⁸Ag, ¹⁰⁹Pd, ^{109m}Pd, ^{114m}In, ¹¹⁵Cd, ¹²¹Sn, ¹²²Sb, ¹²⁷Te, ¹²⁹Te, ¹³³Xe, ¹³⁵Xe, ¹⁴¹Ce, ¹⁴⁷Nd, ¹⁴⁹Nd, ¹³⁵Sm, ^{152m}Eu, ¹⁵⁹Gd, ¹⁶⁹Er, ¹⁵⁵Yb, ¹⁷⁶Lu, ¹⁸⁵W, ¹⁸⁶Re, ¹⁹¹Os, ¹⁹²Ir, ¹⁹⁷Pt, ^{197m}Pt. Hence, to produce particles labelled with the preceding radionuclides through (γ, n) reactions with the present method, the particles to be irradiated should normally comprise as precursor nuclides a stable isotope of the same element as the desired radionuclide, with a mass number superior by one unit to the mass number of the desired radionuclide.

For nuclear medical imaging, particles labelled with positron (β+) emitters are preferred. The (β+ emitting nuclides interact with electrons via an annihilation process, which results in the production of two 0.511 MeV photons that can be detected e.g. by positron emission tomography cameras. A preferred list of suitable radionuclides is the following: ¹¹C, ¹³N, ¹⁵O, ¹⁸F, ³⁰F, ^{34m}Cl, ³⁸K, ⁴⁴Sc, ⁴⁵Ti, ⁴⁹Cr, ⁵³Fe, ⁵⁷Ni, ⁶²Cu, ⁶⁴Cu, ⁶³Zn, ⁸⁸Ga, ⁶⁹Ge, ⁷⁸Sr, ⁸⁴Rb, ⁹⁵Ru, ¹⁰¹Pd, ¹⁰⁶Ag, ¹⁰⁵Cd, ¹¹²ln, ¹²⁰Sb, ¹²⁶I, ¹⁴⁰Pr, ¹⁴¹Nd, ¹⁹⁰ⁿIr, ¹⁹⁶Au. Again, to produce particles labelled with the preceding radionuclides through (γ, n) reactions with the present method, the particles to be irradiated should comprise as precursor nuclide a stable isotope of the same element as the desired radionuclide, with a mass number superior by one unit to the mass number of the desired radionuclide.

Hence, the present method allows production of both β- and β+ emitting radiolabeled particles. As a matter of fact, when preparing particles from a given precursor element and carrier material, the precursor element may include different naturally occurring stable isotopes of this element. As a result, upon irradiation in the photon irradiating field, the radiolabeled particles will emit both β- and β+ radiations. It will thus be appreciated that the present method allows producing radiolabeled particles that can simultaneously be used for therapeutical and imaging purposes, thereby allowing the treatment of tumours and observation of the effect. In this connection, the following elements are preferred as precursors: Ga, Zn, Ge, Br, Kr, Mo, Pd, Cd, Sb, Nd, Er.

The intensity of the laser beam used to produce the irradiating field by interaction with the converting means needs to be of sufficient energy so that the photons, respectively the protons, produced are of sufficient energy to drive the (γ, n) reactions. Preferably, the intensity of the laser is of at least 10¹⁹ W/cm², more preferably about 10²⁰ W/cm² and higher.

During irradiation, the particles are advantageously placed in a container. The container is preferably made of a light element (e.g. aluminium) to limit absorption of the produced radiations by the container itself.

The distribution of the particles in the zones to be treated or analysed depends on the size of the particles. A preferred size range for the particles is between 10 nm and 500 µm, more preferably between 1 and 100 µm, depending on the application. The particles may be of various shapes, although spherical particles are generally preferred.

For internal patient use, the particles should preferably be in a form that is insoluble in *"in vivo"* conditions, i.e. in cellular media. If the precursor element (stable nuclide) to be activated can be elaborated into particles of a chemical form that is insoluble in cellular media, then there is no need for carrier material. However, the stable precursor nuclides are often combined with carrier material to form particles, in particular by encapsulation with insoluble carrier material.

According to another aspect of the present invention, a method for producing radiolabeled particles for internal radiopharmaceutical use comprises a first step of providing particles comprising precursor nuclides and a subsequent step of activating the particles to obtain radiolabeled particles. The activation step is performed using bremstrahlung photons, as explained hereinabove.

In the present method, nuclear reactions required for activation of the particles are induced by the laser-produced irradiating field, thereby eliminating the need for a nuclear reactor. This method avoids one particularly constraining aspect of conventional methods, where radiolabeled particles must be activated in nuclear reactors out of hospitals. Indeed, with the present method, the important activation step of the production of radiolabeled particles can be carried out on the site of use, since today's laser systems are well suited to be installed in hospitals. This has an important impact on the types of radiolabeled particles that can be produced, since it allows production of radiolabeled particles comprising radionuclides having a short half-life time, and which could not be used before in hospitals far away from nuclear reactors.

The method advantageously includes a further step of suspending the radiolabeled particles in an appropriate aqueous media to be injected into a patient's body, e.g. in a tumour and/or in a zone to be analysed.

When producing the particles to be irradiated in a photon field, the precursor nuclides are selected in function of the desired application, as already explained. For cancer therapy, the precursor nuclides used for preparing the particles are preferably stable isotopes of an element that will be converted, through (γ, n) reactions, into beta- emitting radionuclides. For medical nuclear imaging, the precursor nuclides used for preparing the particles are preferably stable isotopes of an element that will be converted, through (γ, n) reactions, into β+ emitting radionuclides. In addition, the particles may comprise as precursor nuclides a mixture of stables isotopes, part of the isotopes being activated into β- emitting radionuclides and part of the isotopes being activated into β+ emitting radionuclides.

Accordingly, the present invention also proposes radiolabeled particle for internal radiopharmaceutical use, such as a nanoparticle or microparticle, comprising both radionuclides emitting β- rays in an energy range suitable for cancer therapy and radionuclides emitting β+ rays in an energy range suitable for medical nuclear imaging. Such a radiolabeled particle containing both positron and negatron emitters allows visualisation of the location of the particle and concurrently provides the therapeutic treatment The radionuclide mixture in the microparticle may comprise β- and β+ emitter radionuclides from a same element, or from different elements. With regard to the types of elements suitable for internal pharmaceutical use, the radionuclides preferably comprises a mixture of β- emitters selected from the list comprising: ⁷⁰Ga, ⁶⁹Zn, ⁷⁵Ge, ⁸⁰Br, , ^{85m}Kr, ⁹⁹Mo, ¹⁰⁹Pd, ¹¹⁵Cd, ¹²²Sb, ¹⁴⁰Nd and ¹⁶⁹Er; and β+ emitters selected from the list comprising: ⁶⁸Ga, ⁶³Zn, ⁶⁹Ge, ⁷⁸Br, ⁷⁹Kr, ⁹¹Mo, ¹⁰¹Pd, ^{105.107}Cd, ¹²⁰Sb, ¹⁴¹Nd and ¹⁶⁵Er.

### BRIEF DESCRIPTION OF THE DRAWING

The present invention will now be described, by way of example, with reference to the accompanying drawing, in which:

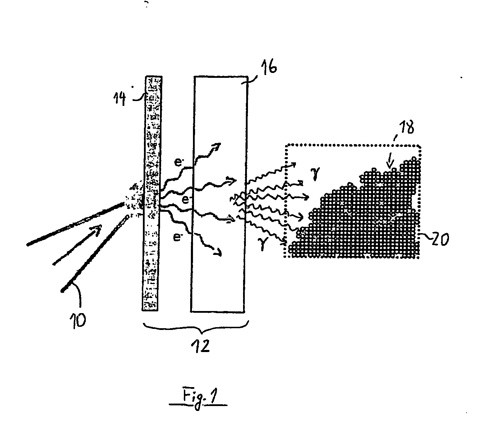

FIG. 1: is a sketch of the experimental set-up used to implement a preferred embodiment of the present method.

### DETAILED DESCRIPTION OF A PREFERRED EMBODIMENT

A preferred embodiment of a method according to the invention will now be described in more detail, wherein radiolabeled particles are produced using an activation step involving photon irradiation. The present method comprises two main steps of: (1) preparing particles comprising precursor nuclides, and (2) irradiating the particles in a laser-produced photon irradiating field to activate them into radiolabeled particles.

### I. Preparation of the particles

Particles for pharmaceutical use are known as microparticles, nanoparticles, microspheres, microcapsules or beads. They are typically of spherical shape and have dimensions (mean diameters) ranging between 10 nm and 500 µm.

For internal patient use, the particles should preferably be in a form that is insoluble in *"in vivo"* conditions, i.e. in cellular media. If particles comprising stable nuclides can be elaborated into particles of a chemical form that is insoluble in cellular media, then there is no need for carrier material. For example, a carrier is not required for insoluble oxides of the lanthanides and of other metals predominantly on the left side of the transition metal block of the periodic table.

However, in many cases the stable radionuclides are combined with carrier material to form particles. In particular, coating and encapsulation with the carrier material can avoid the necessity for insoluble precursor nuclide compounds. Moreover, a carrier material is preferably used for dilution of the radionuclide or density adjustment (particularly to that of the cellular media). The carrier material may be based on polymers, polymeric resins, albumin, or inorganic materials such as e.g. glass. Frequently, microspheres are prepared by producing spheres of the carrier material and combining the radionuclide with the sphere of carrier material e.g. by complexation.

Particle coatings can also be considered for soluble (or even insoluble) materials, whereby the coating should be insoluble and preferably have a thickness between 1 and 100 µm, more preferably below 20 µm.

It will be understood that since a given metal may naturally occur under two or more stable isotopic forms, a particle prepared from this metal (without prior isotopic separation), to comprise as precursor nuclide one of these stable isotopes, may also comprise other stable isotopes of this metal.

A variety of methods have been proposed for producing microspheres and nanoparticles, and will not be discussed in detail herein. Any method allowing to produce particles comprising precursor nuclides optionally combined with carrier material can be used for the preparation of particles for the production of radiolabeled particles according to the present method.

Hence, the term "particles comprising precursor nuclides" herein refers to any particles comprising precursor nuclides, and in particular to: particles of a precursor nuclide in pure or compound form that is insoluble In cellular media; particles consisting of precursor nuclides encapsulated in carrier material; or particles of carrier material to which precursor nuclides are bound.

### II. Activation of the particles

According to the present method, after production of the particles comprising the precursor nuclides, the latter are activated by bremsstrahlung photons generated by interaction of a laser beam onto a metallic target

A preferred experimental set-up for conducting the irradiating step is shown in Fig.1. A high-intensity laser beam 10 is focused onto a converting means generally indicated 12. The converting means 12 itself preferably comprises two target parts, more specifically of two sheets 14, resp. 16, of tantalum with a thickness of 50 µm and 1 mm, respectively. The laser beam 10 impinges onto the first target part 14. The angle of incidence of the laser beam 10 is preferably less than 45° in parallel polarization, as this geometry allows for high absorption of laser light into a plasma. The second target part 16 is placed behind the first target part 14 (with regard to the incident laser beam). The particles comprising the precursor nuclides 18 to be activated are placed in bulk in a container 20, preferably made of aluminium, which is positioned behind the second target part 16.

The incident laser beam 10 is preferably generated by means of a so-called tabletop laser. Preferred laser parameters are the following:
Laser pulse energy: 1 J (1 Joule)
Laser pulse length: 50x10⁻¹⁵ s (50 femtoseconds)
Laser intensity: 10²⁰ W/cm²
Focal spot diameter: 5 µm²
The high-intensity laser beam 10 produces a relativistic plasma on the surface of the first target part 14. Plasma electrons are accelerated to relativistic energies within the intense laser field. These fast electrons impinge on the second target part 16, which serves as an efficient bremsstrahlung converter. As a result, an irradiating field of high-energy bremsstrahlung photons is produced behind the second target part 16 (schematically illustrated by γ waves in Fig.1), whereby the particles 18 are irradiated with these bremsstrahlung photons. With these laser operating parameters, photons having an energy of up to 30 MeV and more can be obtained.

The photons impinging onto the particles induce (γ, n) photonuclear reaction in the precursor nuclides. Indeed, absorption of high-energy electromagnetic radiation in the form of gamma-ray photons causes the nucleus of a precursor nuclide to eject a neutron, resulting in the formation of a radioactive isotope. As a result, radiolabeled particles are obtained.

Regarding more particularly the laser beam parameters, the laser pulse repetition rate should preferably be as high as possible, for productivity reasons. Presently laser technology already permits these goals, but, with the actual developments of the laser technology, it should soon be possible to implement the present method with lasers system having a pulse repetition rate of higher than 10 Hz, higher energy per shot and likely higher intensity.

### III. Choice of the radionuclide

The choice of radionuclide depends on the intended use of the radio-labeled particle. For radiotherapy, the radionuclide must have an appropriate radiation spectrum for treating small to large multiple tumours. Generally, β-emitters are used. For nuclear medical imaging, the radionuclide should be a β+ emitter, so that γ-rays are produced due to annihilation process.

A list of preferred β- emitting nuclides which can be produced by (γ, n) reactions and that are of interest for cancer therapy is given in table 1. These nuclides (second column) can be obtained by irradiating particles comprising as precursor nuclides a stable isotope indicated in the first column (the precursor isotope is underlined; the abundance percentage of this isotope is also indicated in brackets). Half-life, Main β- rays and main γ-rays are also Indicated, with their respective emission probability.

**Table 1. List of β- emitters**

| **Stable isotope precur-sor(s)** | **Nuclide** | **Half-life** | **Main β-(MeV)** | **Main γ** |
|---|---|---|---|---|
| ^{69.71}Ga(40%) | ⁷⁰Ga | 21.14 min | 1.7 (99%) | 1.04 MeV (0.6%) |
| ^{70,72-74,76}Ge (7%) | ⁷⁵Ge | 1.38 h | 1.2 (87%) | 265 keV (13%) |
| ^{79.81}Br (49%)) | ⁸⁰Br | 18 min | 2 (84%) | 612 keV (7%) |
| ^{74,76-78,80,82}Se (9%) | ⁵¹Se | 18.45 min | 1.6 (98%) | 276 keV (0.8%) |
| | ^{81m}Se | 57.8 min | 1.2 (0.06%) | 103 keV (13%) |
| ^{78,80,82-84,86}Kr (17%) | ^{85m}Kr | 4.4 h | 0.84 (79%) | 151 keV (75%) |
| ^{85,87}Rb (29%) | ⁸⁸Rb | 18.6 d | 1.7 (91%) | 1.1 MeV (8%) |
| ^{94-98,100}Mo (10%) | ⁹⁹Mo | 2.75 d | 1.2 (82%) | 739 keV (12%) |
| ^{98-102,104}Ru (19%) | ¹⁰³Ru | 39.26 d | 0.225 (90%) | 497 keV (89%) |
| ^{107,109}Ag (48%) | ¹⁰⁸Ag | 2.37 min | 1.6 (96%) | 633 keV (0.2%) |
| ^{102,104-106,108,110}Pd (12%) | ¹⁰⁹Pd | 13.7 d | 1.03 (99%) | 311 keV (0.03%) |
| | ^{109m}Pd | 4.7 min | - | 189 keV (48%) |
| ^{113,116}in (96%) | ^{114m}In | 49.51 d | 0.35 (4%) | 190 keV (15%) |
| ^{106,108,110,116}Cd (7%) | ¹¹⁵Cd | 2.23 d | 1.1 (63%) | 527 keV (27%) |
| ^{112,114-119,122,124}Sn (5%) | ¹²¹Sn | 1.13 d | 0.383 (100%) | - |
| ^{121,123}Sb (43%) | ¹²²Sb | 2.72 d | 1.4 (66%) | 564 keV (70%) |
| ^{120,122-126,128,130}Te (32%) | ¹²⁷Te | 9.35 h | 0.694 (99%) | 498 keV (1%) |
| | ¹²⁹Te | 69 min | 1.5 (89%) | 459 (7%) |
| ^{124,126,128-132,134,136}Xe (10%) | ¹³³Xe | 5.24 d | 0.346 (99%) | 81 keV (37%) |
| | ¹³⁵Xe | 9.14 h | 0.908 (95%) | 250 keV (90%) |
| ^{136,138,140,142}Ce (11%) | ¹⁴¹Ce | 32.5 d | 0.436 (70%) | 145 keV (48%) |
| ^{142-146,148,150}Nd (5%) | ¹⁴⁷Nd | 10.98 d | 0.804 (81%) | 531 keV (13%) |
| | ¹⁴⁹Nd | 1.73 h | 1.5 (28%) | 211 keV (25%) |
| ^{144,147-150,154}Sm (23%) | ¹⁵³Sm | 1.93 d | 0.713 (43%) | 103 keV (28%) |
| ^{151,153}Eu (53%) | ^{152m} Eu | 9.31 h | 1.9 (68%) | 841 keV (15%) |
| ^{152,154-158,160}Gd (22%) | ¹⁵⁹Gd | 18.5 h | 0.974 (62%) | 363 keV (10%) |
| ^{162.164.166.168.170}Er (15%) | ¹⁶⁹Er | 9.4 d | 0.352 (58%) | - |
| ^{168,170-170,176}Yb (13%) | ¹⁷⁵Vb | 4.2 d | 0.467 (87%) | 396 keV (7%) |
| ^{175,176}Lu (3%) | ^{176m}Lu | 3.74 h | 1.2 (60%) | 88 keV (9%) |
| ^{180,182-184,186}W (28%) | ¹⁸⁵W | 75 d | 0.432 (99%) | 125 keV (0.02%) |
| ^{185,187}Re (63%) | ¹⁸⁶Re | 3.72 d | 1.1 (74%) | 137 keV (8%) |
| ^{184,186-190,192}Os (41%) | ¹⁹¹Os | 15.4 d | 0.141 (100%) | 129 keV (29%) |
| ^{191,193}Ir (63%) | ¹⁹²Ir | 73.83 d | 0.672 (48%) | 316 keV (83%) |
| ^{190,192,194-196,198}Pt (7%) | ¹⁹⁷Pt | 19.89 h | 0.641 (81%) | 77 keV (17%) |
| | ^{197m}Pt | 1.97 h | 0.709 (3%) | 346 keV (11%) |

A list of preferred β+ emitting nuclides which can be produced by (γ, n) re-actions and that are of interest for medical nuclear imaging is given in table 2. These nuclides (second column) can be obtained by irradiating particles comprising as precursor nuclides a stable isotope indicated in the first column (the abundance percentage of this isotope is also indicated in brackets). Half-life, Main β+ rays (emission probability indicated in brackets) and threshold for the (γ, n) reaction are also indicated.

**Table 2. List of positron β+) emitters**

| **Stable isotope(s) precursor** | **Nuclides** | **Half-life** | **Main β+(MeV)** | **Threshold for (γ,n) reaction** |
|---|---|---|---|---|
| ¹²C (99%) | ¹¹C | 20.4 min | 1.98 (100%) | 18 |
| ¹⁴N (99%) | ¹³N | 10 min | 2.22 (100%) | 15 |
| ¹⁵O (99%) | ¹⁵O | 2 min | 2.75 (100%) | 17 |
| ¹⁹F (100%) | ¹⁸F | 1.8 h | 1.65 (100%) | 10 |
| ³¹P (100%) | ³⁰P | 2.5 min | 4.2 (100%) | 12.3 |
| ³⁵Cl (76%) | ^{34M}Cl | 32 min | 3.5 (28%) 2.3 (24%) | 12.6 |
| ³⁹K (93%) | ³⁸K | 7.64 min | 3.7 (99%) | 13.1 |
| ⁴⁵Sc (100%) | ⁴⁴Sc | 3.93 h | 2.5 (99%) | 11.3 |
| ⁴⁶Ti (8%) | ⁴⁵Ti | 3.1 h | 2.1 (100%) | 13.2 |
| ⁵⁰Cr (4%) | ⁴⁹Cr | 42 min | 2.47 (52%) 2.54 (37%) | 13.0 |
| ⁵⁴Fe (6%) | ⁵³Fe | 8.5 m | 3.74 (58%) 3.37 (39%) | 13.4 |
| ⁵⁸Ni (68%) | ⁵⁷Ni | 1.5 d | 1.87 (34%) | 12.2 |
| ⁶³Cu (69%) | ⁶²Cu | 9.7 min | 3.9 (99%) | 10.9 |
| ⁶³Cu (31%) | ⁶⁴Cu | 12.7 h | 0.578 (40%) | 9.9 |
| ⁶⁴Zn (49%) | ⁶³Zn | 38.5 min | 3.37 (84%) | 11.9 |
| ⁶⁹Ga (60%) | ⁶⁸Ga | 1.1 h | 2.92 (97%) | |
| ⁷⁰Ge (21%) | ⁶⁹Ge | 1.6 d | 2.23 (31%) | 11.5 |
| ⁷⁹Br (49%) | ⁷⁸Br | 6.46 min | 3.6 (86%) | |
| ⁸⁵Rb (72%) | ⁸⁴Rb | 33 d | 1.8 (68%) | 10.5 |
| ⁹⁸Ru (6%) | ⁹⁵Ru | 1.6 h | 2.26 (37%) 1.13 (25%) | |
| ¹⁰²Pd (1%a) | ¹⁰¹Pd | 8.5 h | 1.8 (57%) 1.2 (22%) | 10.6 |
| ¹⁰⁷Ag (52%) | ¹⁰⁶Ag | 24 min | 2.97(83%) 2.45 (16%) | 9.6 |
| ¹⁰⁶Cd (1.2%) | ¹⁰⁵Cd | 55 min | 2.71 (54%) 1.10 (50%) | 10.6 |
| ¹¹³In (4%) | ¹¹²In | 15 min | | 9.4 |
| ¹²¹Sb (57%) | ¹²⁰Sb | 15.9 min | 2.68 (98%) | 9.2 |
| ¹²⁵I (100) | ¹²⁶I | 13 d | 0.826 (32%) | 9.1 |
| ¹⁴¹Pr (100%) | ¹⁴⁰Pr | 3.4 m | 3.39 (99%) | 9.4 |
| ¹⁴²Nd (27%) | ¹⁴¹Nd | 2.5 h | 1.81 (98%) | 9.8 |
| ¹⁹¹Ir (37%) | ¹⁹⁰ⁿIr | 3.1 h | 0.470 (94%) | |
| ¹⁹⁷AU (100%) | ¹⁹⁶AU | 6.18 d | 1.1 (67%) | 8.1 |

The present method thus permits the production of radiolabeled particles comprising a radionuclide listed in table 1 or 2. Such a radiolabeled particle is obtained by: (1) preparing a particle comprising a stable precursor nuclide indicated in the first column of the tables (generally having a mass number superior by one unit to the radionuclide); and (2) activating the particle in a photon irradiating field produced by interaction of a laser beam onto a solid target.

It will be noted that when irradiating two stable isotopes of a same element with photons, one isotope may be converted into a β- emitting radionuclide whereas the other isotope is converted into a β+ emitting radionuclide.

Hence, when the particles comprise a mixture of such precursor isotopes, the present method allows production of radiolabeled particles that concurrently emit β+ and β- rays, so that they can be used for both cancer therapy and medical nuclear imaging (diagnosis or spatial localisation).

A list of preferred elements that allow simultaneous production of β- and β+ emitters through (y, n) reactions is given in table 3. The first column gives two precursor isotopes of a same element (abundance % in brackets), and the radionuclides produced by (γ, n) reactions are given in the second column. The half-lives of these radionuclides are indicated in the last column.

**Table 3. List of β+ and β-emitters that can simultaneously be produced from different isotopes of a same element with the present method.**

| **Stable Isotope(s) precursor** | **Radionuclides** | **Half-life** |
|---|---|---|
| ⁶⁹Ga (60%), | ⁶⁸Ga (β+), | 1.1 h |
| ⁷¹Ga (40%) | ⁷⁰Ga (β-) | 21 min |
| ⁵⁴Zn (49%), | ⁶³Zn (β+), | 38 min |
| ⁷⁰Zn (0.6%) | ⁶⁹Zn (β-) | 56 min |
| ⁷⁰Ge (21%), | ⁶⁹Ge (β+), | 1.6 d |
| ⁷⁶Ge (7%) | ⁷⁵Ge (β-) | 1.4 h |
| ⁷⁹Br (51%), | ⁷⁸Br (β+), | 6 min |
| ⁸¹Sr (49%) | ⁸⁰Br (β-) | 18 min |
| ⁸⁰Kr (2%), | ⁷⁹Kr (β+), | 1.5 d |
| ⁸⁶Kr (17%) | ^{85m}Kr (β-) | 4.5 h |
| ⁹²Mo (15%), | ⁹¹Mo (β+), | 15.5 m |
| ¹⁰⁰Mo (10%) | ⁹⁹Mo (β-) | 2.75 d |
| ¹⁰²Pd (1%) | ¹⁰¹Pd (β+) | 8.5 h |
| ¹¹⁰Pd (13%) | ¹⁰⁹Pd (β-) | 13.7 h |
| ^{106,108}Cd (1%) | ^{105,107}Cd (β+) | 55 m, 6.5 h |
| ¹¹⁶Cd (7.5%) | ¹¹⁵Cd (β-) | 2.2 d |
| ¹²¹Sb (57%) | ¹²⁰Sb (β+) | 15.9 min |
| ¹²³Sb (43%) | ¹²²Sb (β-) | 2.7 d |
| ¹⁴²Nd (27%) | ¹⁴¹Nd (β+) | 2.5 h |
| ¹⁵⁰Nd (6%) | ¹⁴⁹Nd (β-) | 1.7 h |
| ¹⁶⁶Er (34%) | ¹⁶⁵Er (β+) | 10.4 h |
| ¹⁷⁰Er (15%) | ¹⁶⁹Er (β-) | 9.4 d |

The particle produced according to the present method may thus comprise a combination of two different radionuclides (from the same or different elements); one radionuclide for producing an optimum p- radiation for therapy and the other one for producing the optimum β+ radiation for diagnosis or spatial location. This can be easily achieved by preparing the particles from the two appropriate kinds of precursor nuclides.

## Claims

1. A radiolabeled particle for internal radiopharmaceutical use, such as a nanoparticle or microparticle, comprising both β- emitting radionuclides suitable for cancer therapy and β+ emitting radionuclides suitable for medical nuclear imaging.

2. The radiolabeled particle according to claim 1, comprising a β- emitting radionuclide selected from the list comprising; ⁷⁰Ga, ⁶⁹Zn, ⁷⁵Ge, ⁸⁰Br, ^{85m}Kr, ⁹⁹Mo, ¹⁰⁹Pd, ¹¹⁵Cd, ¹²²Sb, ¹⁴⁹Nd and ¹⁶⁹Er; and a p+ emitting radionuclide selected from the list comprising ⁶⁸Ga, ⁶³Zn, ⁶⁹Ge, ⁷⁸Br, ⁷⁹Kr, ⁹¹Mo, ¹⁰¹Pd, ^{105,107}Cd, ¹²⁰Sb, ¹⁴¹Nd and ¹⁶⁵Er.

3. The radiolabeled particle according to claim 1, comprising a couple of β-and β+ emitting radionucléides from the same element.

4. The radiolabeled particle according to claim 1, 2 or 3, wherein the dimensions of said particles are in the range of 10 nm to 500 µm, preferably of 1 to 100 µm.

5. The radiolabeled particle according to any one of claims 1 to 4, wherein said radionuclides are combined with carrier material.

6. The radiolabeled particle according to any one of claims 1 to 5, wherein said particles are insoluble in cellular media..

7. A method for activating micro- and/or nanoparticles for internal radiopharmaceutical use, said particles comprising precursor nuclides to be activated,
**characterised by**
directing a high-intensity laser beam onto a converting means to produce an irradiating field of bremsstrahlung photon; and
irradiating said particles comprising precursor nuclides in said irradiating field of bremsstrahlung photon to activate said precursor nuclides, thereby obtaining radiolabeled particles.

8. The method according to claim 7, **characterised in that** said converting means includes a metallic target.

9. The method according to claim 8, **characterised in that** said converting means comprises a first target part on which said laser beam is focused and a second target part behind the first target part that acts as a bremsstrahlung converter.

10. The method according to claim 8 or 9, **characterised in that** said metallic target, respectively target parts, are made of tantalum, tungsten, platinum or copper.

11. The method according to any one of claims 7 to 10, **characterised in that** each particle comprises as precursor nuclide a stable isotope selected of an element selected from the group comprising Ag, Au, Br, C, Cd, Ce, Cl, Cr, Cu, Er, Eu, F, Fe, Ga, Gd, Ge, I, In, Ir, K, Kr, Lu, Mo, N, Nd, Ni, O, Os, P, Pd, Pr, Pt, Rb, Re, Ru, Sb, Sc, Se, Sm, Sn, Te, Ti, W, Xe, Yb, Zn, or combinations thereof.

12. The method according to any one of claims 7 to 11, **characterised in that** said activated radiolabeled particles comprise radionuclides selected from the group comprising: ⁷⁰Ga, ⁷⁵Ge, ⁸⁰Br, ⁸¹Se, ^{81m}Se, ^{85m}Kr, ⁸⁶Rb, ⁹⁹Mo, ¹⁰³Ru, ¹⁰⁸Ag, ¹⁰⁹Pd, ^{109m}Pd, ^{114m}In, ¹¹⁵Cd, ¹²¹Sn, ¹²²Sb, ¹²⁷Te, ¹²⁹Te, ¹³³Xe, ¹³⁵Xe, ¹⁴¹Ce, ¹⁴⁷Nd, ¹⁴⁹Nd, ¹⁵³Sm, ^{152m}Eu, ¹⁵⁹Gd, ¹⁶⁹Er, ¹⁷⁵Yb, ^{176m}Lu, ¹⁸⁵W, ¹⁸⁶Re, ¹⁹¹Os, ¹⁹²Ir, ¹⁹⁷Pt, ^{197m}Pt, ¹¹C, ¹³N, ¹⁵O, ¹⁸F, ³⁰P, ^{34m}Cl, ³⁸K, ⁴⁴Sc, ⁴⁵Ti, ⁴⁹Cr, ⁵⁹Fe, ⁵⁷Ni, ⁶²Cu, ⁶⁴Cu, ⁶³Zn, ⁶⁸Ga, ⁶⁹Ge, ⁷⁸Br, ⁸⁴Rb, ⁹⁵Ru, ¹⁰¹Pd, ¹⁰⁶Ag, ¹⁰⁵Cd, ¹¹²In, ¹²⁰Sb, ¹²⁸I, ¹⁴⁰Pr, ¹⁴¹Nd, ¹⁹⁰ⁿIr, ¹⁹⁶Au, or combinations thereof,

13. The method according to any one of claims 7 to 12, **characterised in that** said laser beam impinging onto said converting means has an intensity of at least 10¹⁹ W/cm², preferably of about 10²⁰ W/cm² and above.

14. The method according to any one of claims 7 to 13, **characterised in that** said particles are in a chemical form that is insoluble in cellular media.

15. The method according to any one of claims 7 to 14, **characterised in that** each of said particles to be activated are particles of a stable isotope in a chemical form that is insoluble in cellular media.

16. The method according to any one of claims 7 to 15, **characterised in that** said particles comprising precursor nuclides are formed by combining precursor nuclides with carrier material.

17. The method according to any one of claims 7 to 16, **characterised in that** each of said particles comprises a stable precursor nuclide that will be activated into a β- emitting radionuclide as well as another stable precursor nuclide that will be activated into a β+ emitting radionuclide.

18. The method according to claim 17, **characterised in that** each particle to be activate comprises a precursor nuclide selected from the list comprising: ⁷¹Ga, ⁷⁰Zn , ⁷⁶Ge, ⁸¹Br, ⁸⁸Kr, ¹⁰⁰Mo, ¹¹⁰Pd, ¹¹⁶Cd, ¹²³Sb, ¹⁵⁰Nd and ¹⁷⁰Er; and a precursor nuclide selected from the list comprising: ⁶⁹Ga, ⁶⁴Zn, ⁷⁰Ge, ⁷⁹Br, ⁸⁰Kr, ⁹²Mo, ¹⁰²Pd, ^{106,08}Cd, ¹²¹Sb, ¹⁴²Nd, and ¹⁶⁶Er

19. The method according to claim 17, **characterised in that** each particle to be activated comprise precursor nuclides couples selected from the following list: (⁶⁹Ga; ⁷¹Ga), (⁶⁴Zn; ⁷⁰Zn), (⁷⁰Ge; ⁷⁸Ge), (⁷⁹Br, ⁸¹Br), (⁸⁰Kr, ⁸⁶Kr), (⁹²Mo, ¹⁰⁰Mo), (¹⁰²Pd, ¹¹⁰Pd), (^{106,108}Cd, ¹¹⁶Cd), (¹²¹Sb, ¹²³Sb), (¹⁴²Nd, ¹⁵⁰Nd) and (¹⁶⁶Er, ¹⁷⁰Er).

20. The method according to any one of claims 7 to 19, **characterised in that** the dimensions of said particles are in the range of 10 nm to 500 µm, preferably of 1 to 100 µm.

21. The method according to any one of claims 7 to 20, **characterised in that** said particles comprising precursor nuclides are placed in a container during irradiation.

22. The method according to any one of claims 7 to 21, **characterised in that** it is carried out on the site of use of said radiolabeled particles.

23. A method for producing radiolabeled particles for internal radiopharmaceutical use, comprising the steps of:
providing particles comprising precursor nuclides; and
activating said particles to obtain radiolabled particles;
**characterized in that** said activating step is carried out according to the method as claimed in any one of claims 7 to 22.

24. The method according to claim 23, **characterised by** the step of suspending the radiolabeled particles in an appropriate aqueous media for injection into a patient's body.

25. The particle according to any one of claims 1 to 6 for use as a medicament.

## Patentansprüche

1. Radioaktiv markiertes Partikel zur inneren radiopharmazeutischen Verwendung, beispielsweise als Nanopartikel oder Mikropartikel, umfassend sowohl für die Krebstherapie geeignete β⁻-strahlende Radionuklide als auch für die nuklearmedizinische Bildgebung geeignete β⁺-strahlende Radionuklide.

2. Radioaktiv markiertes Partikel nach Anspruch 1, umfassend ein β⁻- strahlendes Radionuklid, das ausgewählt ist aus der Liste umfassend: ⁷⁰Ga, ⁶⁹Zn, ⁷⁵Ge, ⁸⁰Br, ^{85m}Kr, ⁹⁹Mo, ¹⁰⁹Pd, ¹¹⁵Cd, ¹²²Sb, ¹⁴⁹Nd und ¹⁶⁹Er; und ein β⁺-strahlendes Radionuklid, das ausgewählt ist aus der Liste umfassend: ⁶⁸Ga, ⁶³Zn, ⁶⁹Ge, ⁷⁸Br, ⁷⁹Kr, ⁹¹Mo, ¹⁰¹Pd, ^{105,107}Cd, ¹²⁰Sb, ¹⁴¹Nd und ¹⁶⁵Er.

3. Radioaktiv markiertes Partikel nach Anspruch 1, umfassend ein Paar von β⁻- und β⁺-strahlenden Radionukliden von demselben Element.

4. Radioaktiv markiertes Partikel nach Anspruch 1, 2 oder 3, wobei die Größen der Partikel im Bereich von 10 nm bis 500 µm, vorzugsweise von 1 bis 100 µm, liegen.

5. Radioaktiv markiertes Partikel nach irgendeinem der Ansprüche 1 bis 4, wobei die Radionuklide mit Trägermaterial kombiniert sind.

6. Radioaktiv markiertes Partikel nach irgendeinem der Ansprüche 1 bis 5, wobei die Partikel in Zellmedien unlöslich sind.

7. Verfahren zur Aktivierung von Mikro- und/oder Nanopartikeln zur inneren radiopharmazeutischen Verwendung, wobei die Partikel zu aktivierende Vorläufernuklide umfassen,
**gekennzeichnet durch**
Richten eines hochintensiven Laserstrahls auf ein Umwandlungsmittel, um ein Bremsstrahlungsphoton-Bestrahlungsfeld zu erzeugen; und
Bestrahlen der Partikel umfassend Vorläufernuklide im Bremsstrahlungsphoton-Bestrahlungsfeld, um die Vorläufernuklide zu aktivieren, wodurch radioaktiv markierte Partikel erhalten werden.

8. Verfahren nach Anspruch 7, **dadurch gekennzeichnet, dass** das Umwandlungsmittel ein Metalltarget ist.

9. Verfahren nach Anspruch 8, **dadurch gekennzeichnet, dass** das Umwandlungsmittel Folgendes umfasst: einen ersten Targetteil, auf den der Laserstrahl fokussiert wird, und einen zweiten Targetteil hinter dem ersten Targetteil, der als Bremsstrahlungskonverter wirkt.

10. Verfahren nach Anspruch 8 oder 9, **dadurch gekennzeichnet, dass** das Metalltarget bzw. die Targetteile aus Tantal, Wolfram, Platin oder Kupfer bestehen.

11. Verfahren nach irgendeinem der Ansprüche 7 bis 10, **dadurch gekennzeichnet, dass** jedes Partikel als Vorläufernuklid ein stabiles Isotop umfasst, das aus einem Element ausgewählt ist, das ausgewählt ist aus der Gruppe umfassend: Ag, Au, Br, C, Cd, Ce, Cl, Cr, Cu, Er, Eu, F, Fe, Ga, Gd, Ge, I, In, Ir, K, Kr, Lu, Mo, N, Nd, Ni, O, Os, P, Pd, Pr, Pt, Rb, Re, Ru, Sb, Sc, Se, Sm, Sn, Te, Ti, W, Xe, Yb, Zn oder Kombinationen davon.

12. Verfahren nach irgendeinem der Ansprüche 7 bis 11, **dadurch gekennzeichnet, dass** die aktivierten radioaktiv markierten Partikel Radionuklide umfassen, die ausgewählt sind aus der Gruppe umfassend: ⁷⁰Ga, ⁷⁵Ge, ⁸⁰Br, ⁸¹Se, ^{81m}Se, ^{85m}Kr, ⁸⁶Rb, ⁹⁹Mo, ¹⁰³Ru, ¹⁰⁸Ag, ¹⁰⁹Pd, ^{109m}Pd, ^{114m}In, ¹¹⁵Cd, ¹²¹Sn, ¹²²Sb, ¹²⁷Te, ¹²⁹Te, ¹³³Xe, ¹³⁵Xe, ¹⁴¹Ce, ¹⁴⁷Nd, ¹⁴⁹Nd, ¹⁵³Sm, ^{152m}Eu, ¹⁵⁹Gd, ¹⁶⁹Er, ¹⁷⁵Yb, ^{176m}Lu, ¹⁸⁵W, ¹⁸⁶Re, ¹⁹¹Os, ¹⁹²Ir, ¹⁹⁷Pt, ^{197m}Pt, ¹¹C, ¹³N, ¹⁵O, ¹⁸F, ³⁰P, ^{34m}Cl, ³⁸K, ⁴⁴SC, ⁴⁵Ti, ⁴⁹Cr, ⁵³Fe, ⁵⁷Ni, ⁶²Cu, ⁶⁴Cu, ⁶³Zn, ⁶⁸Ga, ⁶⁹Ge, ⁷⁸Br, ⁸⁴Rb, ⁹⁵Ru, ¹⁰¹Pd, ¹⁰⁶Ag, ¹⁰⁵Cd, ¹¹²In, ¹²⁰Sb, ¹²⁶I, ⁶³Zn, ⁶⁸Ga, ⁶⁹Ge, ⁷⁸Br, ⁸⁴Rb, ⁹⁵Ru, ¹⁰¹Pd, ¹⁰⁶Ag, ¹⁰⁵Cd, ¹¹²In, ¹²⁰Sb, ¹²⁶I, ¹⁴⁰Pr, ¹⁴¹Nd, ¹⁹⁰ⁿIr, ¹⁹⁶Au oder Kombinationen davon.

13. Verfahren nach irgendeinem der Ansprüche 7 bis 12, **dadurch gekennzeichnet, dass** der auf das Umwandlungsmittel auftreffende Laserstrahl eine Intensität von mindestens 10¹⁹ W/cm², vorzugsweise ungefähr 10²⁰ W/cm² und darüber aufweist.

14. Verfahren nach irgendeinem der Ansprüche 7 bis 13, **dadurch gekennzeichnet, dass** die Partikel in einer chemischen Form vorliegen, die in Zellmedien unlöslich ist.

15. Verfahren nach irgendeinem der Ansprüche 7 bis 14, **dadurch gekennzeichnet, dass** die zu aktivierenden Partikel Partikel eines stabilen Isotops in einer chemischen Form sind, die in Zellmedien unlöslich ist.

16. Verfahren nach irgendeinem der Ansprüche 7 bis 15, **dadurch gekennzeichnet, dass** Partikel umfassend Vorläufernuklide durch Kombinieren von Vorläufernukliden mit Trägermaterial gebildet werden.

17. Verfahren nach irgendeinem der Ansprüche 7 bis 16, **dadurch gekennzeichnet, dass** jedes der Partikel Folgendes umfasst: ein stabiles Vorläufernuklid, das zu einem β⁻-strahlenden Radionuklid aktiviert wird, und ein anderes stabiles Vorläufernuklid, das zu einem β⁺-strahlenden Radionuklid aktiviert wird.

18. Verfahren nach Anspruch 17, **dadurch gekennzeichnet, dass** jedes zu aktivierende Partikel Folgendes umfasst: ein Vorläufernuklid, das ausgewählt ist aus der Liste umfassend: ⁷¹Ga, ⁷⁰Zn, ⁷⁶Ge, ⁸¹Sr, ⁸⁶Kr, ¹⁰⁰Mo, ¹¹⁰Pd, ¹¹⁶Cd, ¹²³Sb, ¹⁵⁰Nd und ¹⁷⁰Er; und ein Vorläufernuklid, das ausgewählt ist aus der Liste umfassend: ⁶⁹Ga, ⁶⁴Zn, ⁷⁰Ge, ⁷⁹Br, ⁸⁰Kr, ⁹²Mo, ¹⁰²Pd, ^{106,108}Cd, ¹²¹Sb_{,} ¹⁴²Nd und ¹⁶⁶Er.

19. Verfahren nach Anspruch 17, **dadurch gekennzeichnet, dass** jedes zu aktivierende Partikel Vorläufernuklidpaare umfasst, die aus der folgenden Liste ausgewählt sind: (⁶⁹Ga; ⁷¹Ga), (⁶⁴Zn ; ⁷⁰Zn), (⁷⁰Ge ; ⁷⁶Ge), (¹⁹Br, ⁸¹Br), (⁸⁰Kr, ⁸⁶Kr), (⁹²Mo, ¹⁰⁰Mo), (¹⁰²Pd, ¹¹⁰Pd), (^{106,108}Cd, ¹¹⁶Cd), (¹²¹Sb, ¹²³Sb), (¹⁴²Nd, ¹⁵⁰Nd) und (¹¹⁶Er, ¹⁷⁰Er).

20. Verfahren nach irgendeinem der Ansprüche 7 bis 19, **dadurch gekennzeichnet, dass** die Größen der Partikel im Bereich von 10 nm bis 500 µm, vorzugsweise von 1 bis 100 µm, liegen.

21. Verfahren nach irgendeinem der Ansprüche 7 bis 20, **dadurch gekennzeichnet, dass** die Partikel umfassend Vorläufernuklide während der Bestrahlung in einem Behälter angeordnet sind.

22. Verfahren nach irgendeinem der Ansprüche 7 bis 21, **dadurch gekennzeichnet, dass** es am Ort der Verwendung der radioaktiv markierten Partikel durchgeführt wird.

23. Verfahren zur Herstellung von radioaktiv markierten Partikeln zur inneren radiopharmazeutischen Verwendung, umfassend folgende Schritte:
Bereitstellen von Partikeln umfassend Vorläufernuklide; und
Aktivieren der Partikel, um radioaktiv markierte Partikel zu erhalten;
**dadurch gekennzeichnet, dass** der Aktivierungsschritt nach dem Verfahren nach irgendeinem der Ansprüche 7 bis 22 durchgeführt wird.

24. Verfahren nach Anspruch 23, **gekennzeichnet durch** den Schritt des Suspendierens der radioaktiv markierten Partikel in einem geeigneten wässrigen Medium zur Injektion in den Körper eines Patienten.

25. Partikel nach irgendeinem der Ansprüche 1 bis 6 zur Verwendung als Arzneimittel.

## Revendications

1. Particule radiomarquée pour un usage radiopharmaceutique interne, telle qu'une nanoparticule ou une microparticule, comprenant à la fois des radionucléides à émission de rayonnement β- appropriés à une thérapie contre un cancer et des radionucléides à émission de rayonnement β+ appropriés à une imagerie nucléaire médicale.

2. Particule radiomarquée selon la revendication 1, comprenant un radionucléide à émission de rayonnement β- choisi parmi la liste comprenant : ⁷⁰Ga, ⁸⁹Zn, ⁷⁵Ge, ⁸⁰Br, ^{85m}Kr, ⁹⁹Mo, ¹⁰⁹Pd, ¹¹⁵Cd, ¹²²Sb, ¹⁴⁹Nd et ¹⁶⁹Er ; et un radionucléide à émission de rayonnement β+ choisi parmi la liste comprenant ⁶⁸Ga, ⁶³Zn, ⁶⁹Ge, ⁷⁸Br, ⁷⁹Kr, ⁹¹Mo, ¹⁰¹Pd, ^{105,107}Cd, ¹²⁰Sb, ¹⁴¹Nd et ¹⁶⁵Er.

3. Particule radiomarquée selon la revendication 1, comprenant un couple de radionucléides à émission de rayonnement β- et β+ du même élément.

4. Particule radiomarquée selon la revendication 1, 2 ou 3, dans laquelle les dimensions desdites particules sont dans la plage de 10 nm à 500 µm, préférablement de 1 à 100 µm.

5. Particule radiomarquée selon l'une quelconque des revendications 1 à 4, dans laquelle lesdits radionucléides sont combinés avec un matériau support.

6. Particule radiomarquée selon l'une quelconque des revendications 1 à 5, dans laquelle lesdites particules sont insolubles dans un milieu cellulaire.

7. Procédé d'activation de microparticules et/ou de nanoparticules pour un usage radiopharmaceutique interne, lesdites particules comprenant des nucléides précurseurs destinés à être activés,
**caractérisé par les étapes** :
diriger un faisceau laser haute intensité sur un moyen de conversion pour produire un champ irradiant de photons bremsstrahlung ; et
irradier lesdits particules comprenant des nucléides précurseurs dans ledit champ irradiant de photons bremsstrahlung pour activer lesdits nucléides précurseurs, obtenant ainsi des particules radiomarquées.

8. Procédé selon la revendication 7, **caractérisé en ce que** ledit moyen de conversion inclut une cible métallique.

9. Procédé selon la revendication 8, **caractérisé en ce que** ledit moyen de conversion comprend une première partie cible sur laquelle ledit faisceau laser est focalisé et une deuxième partie cible derrière la première partie cible qui agit comme un convertisseur bremsstrahlung.

10. Procédé selon la revendication 8 ou 9, **caractérisé en ce que** ladite cible métallique, respectivement lesdites parties cibles, sont constituées de tantale, de tungstène, de platine ou de cuivre.

11. Procédé selon l'une quelconque des revendications 7 à 10, **caractérisé en ce que** chaque particule comprend comme nucléide précurseur un isotope stable choisi d'un élément choisi parmi le groupe comprenant :
Ag, Au, Br, C, Cd, Ce, Cl, Cr, Cu, Er, Eu, F, Fe, Ga, Gd, Ge, I, In, Ir, K, Kr, Lu, Mo, N, Nd, Ni, O, Os, P, Pd, Pr, Pt, Rb, Re, Ru, Sb, Sc, Se, Sm, Sn, Te, Ti, W, Xe, Yb, Zn, ou des combinaisons de ceux-ci.

12. Procédé selon l'une quelconque des revendications 7 à 11, **caractérisé en ce que** lesdites particules radiomarquées activées comprennent des radionucléides choisis parmi le groupe comprenant : ⁷⁰Ga, ⁷⁵Ge, ⁸⁰Br, ⁸¹Se, ^{81m}Se, ^{85m}Kr, ⁸⁶Rb, ⁹⁹Mo, ¹⁰³Ru, ¹⁰⁸Ag, ¹⁰⁹Pd, ^{109m}Pd, ^{114m}In, ¹¹⁵Cd ¹²¹Sn, ¹²²Sb, ¹²⁷Te, ¹²⁹Te, ¹³³Xe ¹³⁵Xe, ¹⁴¹Ce, ¹⁴⁷Nd, ¹⁴⁹Nd, ¹⁵³Sm, ^{152m}Eu, ¹⁵⁹Gd, ¹⁶⁹Er, ¹⁷⁵Yb, ^{176m}Lu, ¹⁸⁵W, ¹⁸⁶Re, ¹⁹¹Os, ¹⁹²Ir, ¹⁹⁷Pt, ^{197m}Pt, ¹¹C, ¹³N, ¹⁵O, ¹⁸F, ³⁰P, ^{34m}Cl, ³⁸K, ⁴⁴Sc, ⁴⁵Ti, ⁴⁹Cr, ⁵³Fe, ⁵⁷Ni, ⁶²Cu, ⁶⁴Cu, ⁶³Zn, ⁶⁸Ga, ⁶⁵Ge, ⁷⁸Br, ⁸⁴Rb, ⁹⁵Ru, ¹⁰¹Pd, ¹⁰⁶Ag, ¹⁰⁵Cd, ¹¹²In, ¹²⁰Sb, ¹²⁶I, ¹⁴⁰Pr, ¹⁴¹Nd, ¹⁹⁰ⁿIr, ¹⁹⁶Au, ou des combinaisons de ceux-ci.

13. Procédé selon l'une quelconque des revendications 7 à 12, **caractérisé en ce que** ledit faisceau laser venant frapper ledit moyen de conversion a une intensité d'au moins 10¹⁹ W/cm², préférablement d'environ 10²⁰ W/cm² et plus.

14. Procédé selon l'une quelconque des revendications 7 à 13, **caractérisé en ce que** lesdites particules sont sous une forme chimique qui est insoluble dans un milieu cellulaire.

15. Procédé selon l'une quelconque des revendications 7 à 14, **caractérisé en ce que** chacune desdites particules destinées à être activées sont des particules d'un isotope stable sous une forme chimique qui est insoluble dans un milieu cellulaire.

16. Procédé selon l'une quelconque des revendications 7 à 15, **caractérisé en ce que** lesdites particules comprenant des nucléides précurseurs sont formées par combinaison de nucléides précurseurs avec un matériau support.

17. Procédé selon l'une quelconque des revendications 7 à 16, **caractérisé en ce que** chacune desdites particules comprend un nucléide précurseur stable qui sera activé en un radionucléide à émission de rayonnement β-, ainsi qu'un autre nucléide précurseur stable qui sera activé en un radionucléide à émission de rayonnement β+.

18. Procédé selon la revendication 17, **caractérisé en ce que** chaque particule destinée à être activée comprend un nucléide précurseur choisi parmi la liste comprenant : ⁷¹Ga, ⁷⁰Zn, ⁷⁶Ge, ⁸¹Br, ⁸⁶Kr, ¹⁰⁰Mo, ¹¹⁰Pd, ¹¹⁶Cd, ¹²³Sb, ¹⁵⁰Nd et ¹⁷⁰Er ; et un radionucléide précurseur choisi parmi la liste comprenant : ⁶⁹Ga, ⁶⁴Zn, ⁷⁰Ge, ⁷⁹Br, ⁸⁰Kr, ⁹²Mo, ¹⁰²Pd, ^{106,108}Cd, ¹²¹Sb, ¹⁴²Nd et ¹⁶⁶Er.

19. Procédé selon la revendication 17, **caractérisé en ce que** chaque particule destinée à être activée comprend des couples de nucléides précurseurs choisis parmi la liste suivante : (⁶⁹Ga ; ⁷¹Ga), (⁶⁴Zn ; ⁷⁰Zn), (⁷⁰Ge ; ⁷⁶Ge), (⁷⁹Br ; ⁸¹Br), (⁸⁰Kr; ⁸⁶Kr), (⁹²Mo; ¹⁰⁰Mo), (¹⁰²Pd ¹¹⁰Pd), (^{106,108}Cd; ¹¹⁶Cd), (¹²¹Sb ; ¹²³Sb), (⁴²Nd ; ¹⁵⁰Nd) et (⁶⁶Er ; ¹⁷⁰Er).

20. Procédé selon l'une quelconque des revendications 7 à 19, **caractérisé en ce que** les dimensions desdites particules sont dans la plage de 10 nm à 500 µm, préférablement de 1 à 100 µm.

21. Procédé selon l'une quelconque des revendications 7 à 20, **caractérisé en ce que** lesdites particules comprenant des nucléides précurseurs sont placées dans un récipient pendant l'irradiation.

22. Procédé selon l'une quelconque des revendications 7 à 21, **caractérisé en ce qu'**il est mis en oeuvre sur le site d'utilisation desdites particules radiomarquées.

23. Procédé de production de particules radiomarquées pour un usage radiopharmaceutique interne, comprenant les étapes de :
prévision de particules comprenant des nucléides précurseurs ; et
activation desdites particules pour obtenir des particules radiomarquées ; **caractérisé en ce que** ladite étape d'activation est mise en oeuvre conformément au procédé selon l'une quelconque des revendications 7 à 22.

24. Procédé selon la revendication 23, **caractérisé par** l'étape de mise en suspension des particules radiomarquées dans un milieu aqueux approprié pour une injection dans le corps d'un patient.

25. Particule selon l'une quelconque des revendications 1 à 6 destinée à une utilisation comme un médicament.
